# EUROPÄISCHE PATENTANMELDUNG

(11) **EP 4 494 546 A1**
(43) Veröffentlichungstag der Anmeldung: **22.01.2025**
(21) Anmeldenummer: 23186732.6
(22) Anmeldetag: 20.07.2023
(51) Int. Cl.: A61B 5/00

(54) **ORALES SENSORSYSTEM**

(71) Anmelder: Ivoclar Vivadent AG, 9494 Schaan (LI)
(72) Erfinder: KUHN, Marvin, 9494 Schaan (LI); SKAWRAN, Alexander Albert, 7320 Sargans, Zürich (CH); NIEDRIG, Christian, 9478 Azmoos (CH); GROSSE-HONEBRINK, Alexander, 9463 Oberriet (CH); GEISTER, Florian, 4054 Basel (CH); GLASER, Nicolas, 4125 Riehen (CH)
(74) Vertreter: Baldus, Oliver

(57) **Zusammenfassung**

Die vorliegende Erfindung betrifft ein orales Sensorsystem (100), mit einem Gehäuse (101) mit einer elektrischen Schaltung (103) zum Auswerten von Messsignalen; und einer auswechselbaren Kontaktvorrichtung (105) für die elektrische Schaltung (103), die flüssigkeitsdicht mit dem Gehäuse (101) gekoppelt ist.

## Beschreibung

Die vorliegende Erfindung betrifft ein orales Sensorsystem.

Derzeitige orale Sensorsysteme sind aufwändig in einer Handhabung, da diese zu groß sind oder Zusatzwerkzeuge benötigen. Eine eingegossene Elektrik ist nur zur Einmalverwendung geeignet.

Es ist die technische Aufgabe der vorliegenden Erfindung, ein effizientes und modulares orales Sensorsystem mit einer leichten Handhabung bereitzustellen.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein orales Sensorsystem gelöst, mit einem Gehäuse mit einer elektrischen Schaltung zum Auswerten von Messsignalen; und einer auswechselbaren Kontaktvorrichtung für die elektrische Schaltung, die flüssigkeitsdicht mit dem Gehäuse gekoppelt ist. Das orale Sensorsystem dient zum Ermitteln von Messdaten innerhalb eines Mundraumes eines Patienten. Die Kontaktvorrichtung umfasst beispielsweise einen biochemischen Sensor. Die Kontaktvorrichtung umfasst beispielsweise eine gedruckte Leiterplatte oder eine siebbedruckte Folie.

In einer technisch vorteilhaften Ausführungsform des oralen Sensorsystems umfasst die auswechselbare Kontaktvorrichtung eine klebende und/oder aufquellende Schicht zur flüssigkeitsdichten Kopplung mit dem Gehäuse. Dadurch wird beispielsweise der technische Vorteil erreicht, dass eine gute Abdichtung zwischen dem Gehäuse und der Kontaktvorrichtung entsteht.

In einer weiteren technisch vorteilhaften Ausführungsform des oralen Sensorsystems umfasst die klebende und/oder aufquellende Schicht Hydrokolloide. Dadurch wird beispielsweise der technische Vorteil erreicht, dass sich die Abdichtung bei Kontakt mit Feuchtigkeit verbessert.

In einer weiteren technisch vorteilhaften Ausführungsform des oralen Sensorsystems ist die klebende und/oder aufquellende Schicht um die Kontaktflächen herum auf der Kontaktvorrichtung angeordnet. Dadurch wird beispielsweise der technische Vorteil erreicht, dass die Kontaktflächen vor Feuchtigkeit geschützt sind.

In einer weiteren technisch vorteilhaften Ausführungsform des oralen Sensorsystems weist die Kontaktvorrichtung eine Scheibenform, eine Plattenform oder eine Würfelform auf. Die Kontaktvorrichtung kann auch eine andere Form aufweisen, auf der Leiterbahnen angebracht werden können, wie beispielsweise rechteckig oder quadratisch. Dadurch wird beispielsweise der technische Vorteil erreicht, dass platzsparende Kontaktvorrichtungen für eine orale Anwendung verwendet werden.

In einer weiteren technisch vorteilhaften Ausführungsform des oralen Sensorsystems weist die Kontaktvorrichtung auf der einen und/oder anderen Seite Kontaktflächen auf. Dadurch wird beispielsweise der technische Vorteil erreicht, dass die Kontaktvorrichtung ein oder beidseitig oder zur Durchkontaktierung verwendet werden kann.

In einer weiteren technisch vorteilhaften Ausführungsform des oralen Sensorsystems weist das Gehäuse eine Aussparung zum Einsetzen der Kontaktvorrichtung auf. Dadurch wird beispielsweise der technische Vorteil erreicht, dass die Kontaktvorrichtung vom Gehäuse aufgenommen werden kann.

In einer weiteren technisch vorteilhaften Ausführungsform des oralen Sensorsystems umfasst das Gehäuse oder die Kontaktvorrichtung einen Dichtungsring zum Abdichten der Kontaktvorrichtung oder des Gehäuses in der Aussparung. Dadurch wird beispielsweise der technische Vorteil erreicht, dass ein Eindringen von Feuchtigkeit in die Aussparung verhindert wird.

In einer weiteren technisch vorteilhaften Ausführungsform des oralen Sensorsystems ist ein Rand der Kontaktvorrichtung aus Kunststoff gebildet. Der Kunststoff kann ein Elastomer oder Gummi umfassen. Dadurch wird beispielsweise der technische Vorteil erreicht, dass die Kontaktvorrichtung auf einfache Weise hergestellt werden kann.

In einer weiteren technisch vorteilhaften Ausführungsform des oralen Sensorsystems weist das Gehäuse und/oder die Kontaktvorrichtung ein Schraubgewinde auf. Dadurch wird beispielsweise der technische Vorteil erreicht, dass die Kontaktvorrichtung fest befestigt werden kann.

In einer weiteren technisch vorteilhaften Ausführungsform des oralen Sensorsystems umfasst die Kontaktvorrichtung eine Sensorbasis, die aus einem Dichtmaterial herausragt. Dadurch wird beispielsweise der technische Vorteil erreicht, dass ein zuverlässiger Kontakt hergestellt werden kann.

In einer weiteren technisch vorteilhaften Ausführungsform des oralen Sensorsystems umfasst das Dichtmaterial eine Aussparung zum Einsetzen eines Gehäuseabschnitts. Dadurch wird beispielsweise der technische Vorteil erreicht, dass die Kontaktvorrichtung auf einfache und schnelle Weise befestigt werden kann.

In einer weiteren technisch vorteilhaften Ausführungsform des oralen Sensorsystems umfasst der Gehäuseabschnitt einen vorstehenden Kragen. Dadurch wird beispielsweise der technische Vorteil erreicht, dass ein Eindringen von Feuchtigkeit verhindert wird.

In einer weiteren technisch vorteilhaften Ausführungsform des oralen Sensorsystems umfasst die Aussparung eine umlaufende Vertiefung zum Aufnehmen des Kragens. Dadurch wird beispielsweise der technische Vorteil erreicht, dass ein Eindringen von Feuchtigkeit noch besser verhindert wird.

In einer weiteren technisch vorteilhaften Ausführungsform des oralen Sensorsystems ist das Dichtmaterial aus Gummi oder Silikon gebildet. Dadurch wird beispielsweise der technische Vorteil erreicht, dass eine gute Dichtwirkung erzielt wird.

Ausführungsbeispiele der Erfindung sind in den Zeichnungen dargestellt und werden im Folgenden näher beschrieben.

Es zeigen:
- Fig. 1: eine perspektivische Ansicht und eine Querschnittsansicht einer Kontaktvorrichtung;
- Fig. 2A: schematische Ansichten einer weiteren Ausführungsform einer Kontaktvorrichtung;
- Fig. 2B: schematische Ansichten einer weiteren Ausführungsform einer Kontaktvorrichtung;
- Fig. 3: schematische Ansichten weiterer Ausführungsformen der Kontaktvorrichtung;
- Fig. 4: eine schematische Ansicht eines weiteren oralen Sensorsystems;
- Fig. 5: eine schematische Ansicht eines weiteren oralen Sensorsystems;
- Fig. 6: eine schematische Ansicht eines weiteren oralen Sensorsystems; und
- Fig. 7: eine schematische Ansicht eines weiteren oralen Sensorsystems.

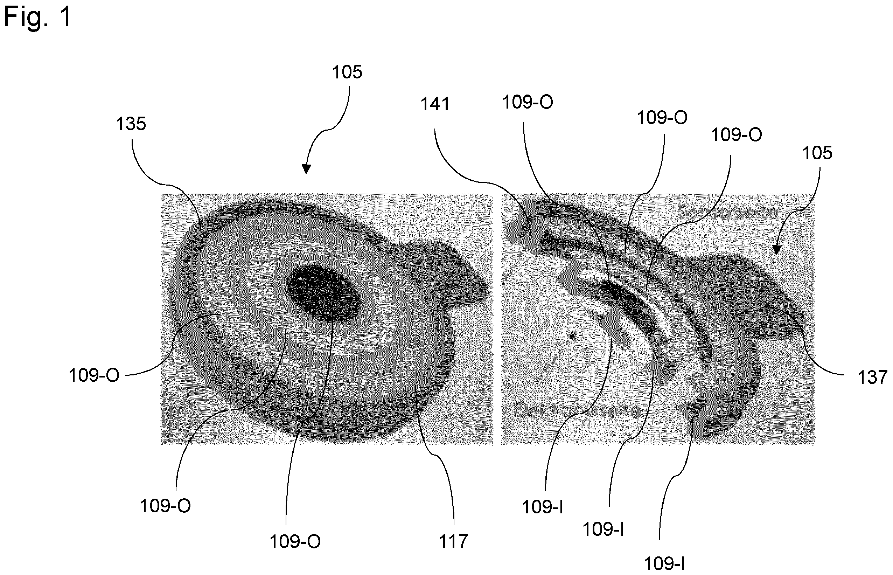

Fig. 1 zeigt eine perspektivische Ansicht und eine Querschnittsansicht einer auswechselbaren und scheibenförmigen Kontaktvorrichtung 105 für ein orales Sensorsystem. Die Kontaktvorrichtung 105 ist beispielsweise ein auswechselbares Kontaktelement. Die Kontaktvorrichtung 105 kann beispielsweise einen biochemischen Sensor für orale Anwendungen umfassen. Das orale Sensorsystem dient zum Erhalten von Messdaten aus dem Mund eines Patienten, wie beispielsweise Laktat- oder pH-Werte.

Die Kontaktvorrichtung 105 dient dabei zum Herstellen eines elektrischen Kontaktes zwischen einem Gehäuse, in dem eine elektrische Schaltung zum Auswerten von Messsignalen angeordnet ist, und einer Kontaktfläche 109-O der Kontaktvorrichtung 105, die in Kontakt mit der oralen Umgebung steht. Die elektrische Schaltung erzeugt geeignete Ströme und Spannungen, die über die Kontaktvorrichtung 105 an die Umgebung abgegeben werden. Durch die auswechselbare Kontaktvorrichtung 105 wird ein reversibles Sensorverschlusssystem zur wasserdichten Anbringung einer Messelektrode an die elektrische Schaltung realisiert.

Die Kontaktvorrichtung 105 weist hierzu an einer Außenseite ringförmige Kontaktflächen 109-O auf, die gegenseitig voneinander getrennt sind. Die Kontaktflächen 109-O an der Außenseite bilden Sensorelektroden und werden über Durchgangskontakte 141 durch die scheiben- oder plattenförmige Kontaktvorrichtung 105 hindurch mit den Kontaktflächen 109-I auf der Innenseite verbunden. Die Kontaktflächen 109-I auf der Innenseite stehen in elektrischem Kontakt mit der elektrischen Schaltung. Ein Träger oder Rand 117 der Kontaktvorrichtung 105 kann aus Kunststoff oder Keramik gebildet sein.

Durch die einsetzbare Kontaktvorrichtung 105 wird ein wasserdichter Verschluss mit einer elektrischen Schaltung zum Auswerten von Messsignalen erreicht. Die einzulegende Kontaktvorrichtung 105 ist hierzu mit einer seitlich umlaufenden Elastomer-Dichtung 135 gefertigt. Die Kontaktvorrichtung 105 wird so mit einem Elastomer umgossen, dass sie wasserdicht in das Gegenstück des Gehäuses eingesetzt werden kann. Die Kontaktvorrichtung 105 umfasst zudem seitlich eine Lasche 137, die das einfache Herausnehmen und Auswechseln ermöglicht.

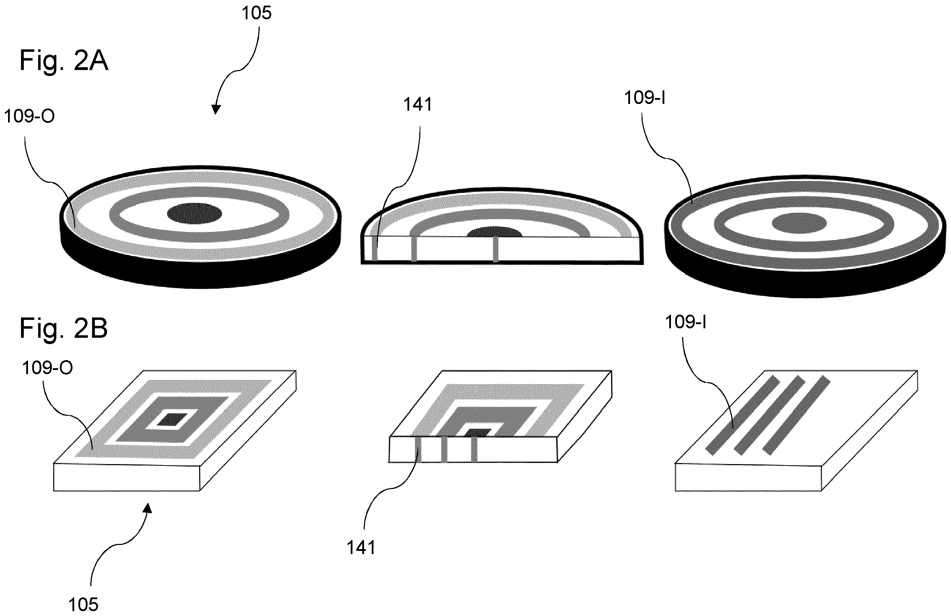

Fig. 2A zeigt schematische Ansichten der scheibenförmigen Kontaktvorrichtung 105. Die Außenseite der Kontaktvorrichtung 105 weist ringförmige Kontaktflächen 109-O auf, die gegenseitig voneinander getrennt sind. Die ringförmige Kontaktflächen 109-O sind über Durchgangskontakte 141 mit ringförmigen Kontaktflächen 109-I auf der Innenseite der Kontaktvorrichtung 105 verbunden.

Fig. 2B zeigt schematische Ansichten einer weiteren Ausführungsform einer rechteckigen Kontaktvorrichtung 105. Die Außenseite der Kontaktvorrichtung 105 weist rechteckförmige Kontaktflächen 109-O auf, die gegenseitig voneinander getrennt sind. Die rechteckförmigen Kontaktflächen 109-O sind über Durchgangskontakte 141 mit streifenförmigen Kontaktflächen 109-I auf der Innenseite der Kontaktvorrichtung 105 verbunden.

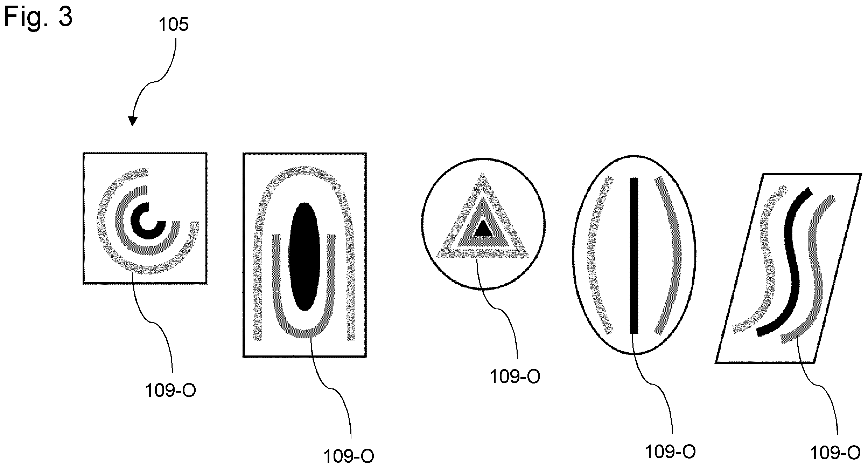

Fig. 3 zeigt schematische Ansichten weiterer Ausführungsformen der Kontaktvorrichtung 105. Die Kontaktvorrichtung 105 kann unterschiedliche Formen aufweisen, wie beispielsweise quadratisch, rechteckig, kreisförmig, oval oder trapezförmig. Auf der Außenseite der Kontaktvorrichtung 105 befinden sich unterschiedlich geformte Kontaktflächen 109-O, wie beispielsweise kreissegmentförmig, u-förmig, dreieckig, gebogen oder s-förmig. Die Formen der Kontaktvorrichtung 105 und die Formen der Kontaktvorrichtung 105 können auf jede Art und Weise kombiniert werden.

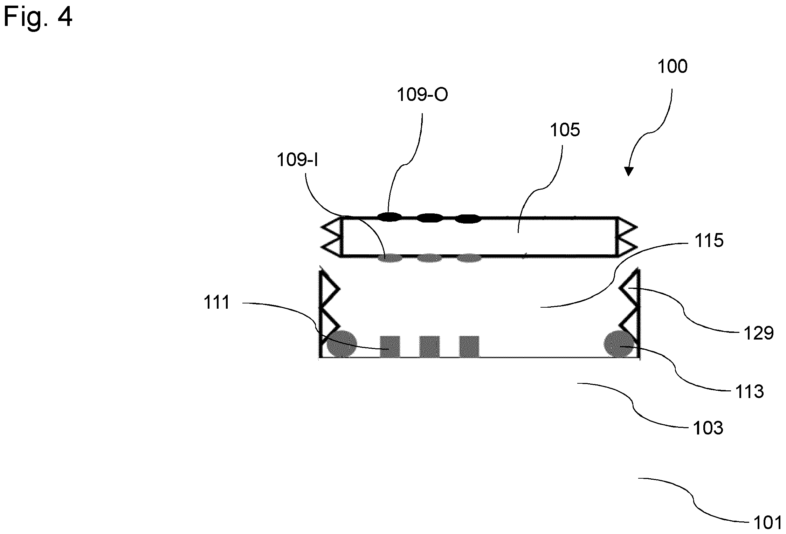

Fig. 4 zeigt eine schematische Ansicht der auswechselbaren Kontaktvorrichtung 105 in dem Gehäuse 101 des oralen Sensorsystems 100. Das Gehäuse 101 umfasst die elektrische Schaltung 103 zum Auswerten von Messsignalen. Die Kontaktvorrichtung 105 wird durch eine Anpressverbindung auf der inneren Seite kontaktiert, so dass eine elektrische Verbindung zwischen den entsprechenden Kontaktflächen 111 der elektrischen Schaltung 103 des Gehäuses 101 und den Kontaktflächen 109-1 der Kontaktvorrichtung 105 entsteht. Die Kontaktflächen 111 des Gehäuses 101 können durch federnde Kontaktstifte gebildet sein. An der äußeren Seite der Kontaktvorrichtung 105 befinden sich die Kontaktflächen 109-O für die elektrische Messung des Sensorsystems 100.

Die Kontaktvorrichtung 105 wird durch einen Dichtungsring 113 wasserdicht an der eingeschlossenen elektrischen Schaltung 103 innerhalb des Gehäuses 101 angebracht. Die scheibenförmige Kontaktvorrichtung 105 wird beispielsweise mittels eines Schraubgewindes 129 in eine zylindrische Aussparung 115 des Gehäuses 101 eingeschraubt. Auf diese Weise wird die Kontaktvorrichtung 105 in dem Gehäuse 101 angeordnet, befestigt und mit den Kontaktflächen 111 kontaktiert.

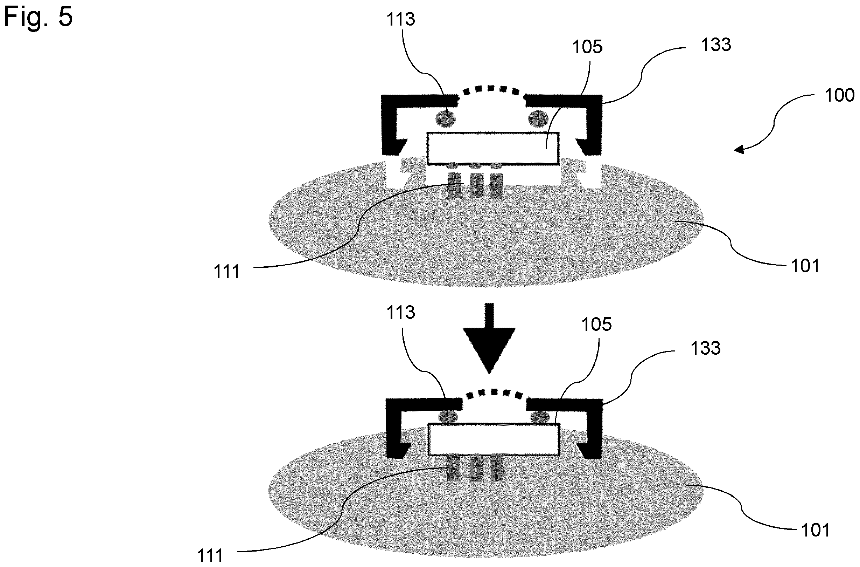

Fig. 5 zeigt eine schematische Ansicht einer weiteren Kontaktvorrichtung 105 des oralen Sensorsystems 100. Die Kontaktvorrichtung 105 wird von einem wiederverwendbaren Klick- oder Schraubmechanismus 133 an der vorgesehenen Verbindungsstelle des Gehäuses 101 gehalten. Der topfförmige Klick- oder Schraubmechanismus 133 umgreift die Kontaktvorrichtung 105 und rastet an dem Gehäuse 101 ein. Damit die Kontaktflächen 109-O in Kontakt mit der Umgebung treten können, umfasst der Klick- oder Schraubmechanismus 133 eine Öffnung. Zwischen der Kontaktvorrichtung 105 und dem Klick- oder Schraubmechanismus 133 ist der Dichtungsring 113 angeordnet.

Die auswechselbare Kontaktvorrichtung 105 kann auf diese Weise wasserdicht an der verschlossenen elektrischen Schaltung 103 angeschlossen werden. Dies ermöglicht eine kompakte Bauform und einfache Handhabung des oralen Sensorsystems 100.

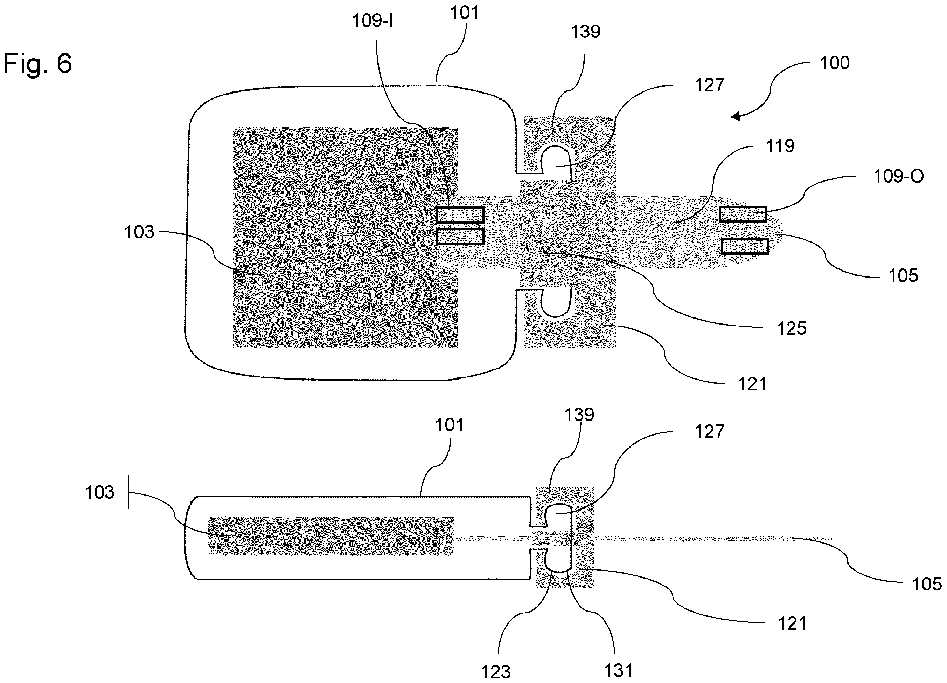

Fig. 6 zeigt eine schematische Ansicht eines weiteren oralen Sensorsystems 100. Die Kontaktvorrichtung 105 ist durch eine Folie gebildet, die in einen Stöpsel 139 eingegossen oder durch diesen hindurchgeführt ist. Die Folie umfasst die Kontaktflächen 109-O. Die Kontaktvorrichtung 105 kann einen biochemischen Sensor für orale Anwendungen umfassen.

Der Stöpsel 139 ist aus gummi- oder silikonartigem Material gebildet und umschließt die folienartige Kontaktvorrichtung 105 seitlich. Die Kontaktvorrichtung 105 ist dadurch so mit einer Dichtung umgeben, dass diese beim Einstecken wasserdicht abgedichtet wird. Die Kontaktflächen 109-I bilden eine Sensorbasis 119, die aus dem Dichtmaterial 121 des Stöpsels 139 herausragt. Das Dichtmaterial 121 umfasst zudem eine Aussparung 123 zum Einsetzen eines Gehäuseabschnitts 125. Die Aussparung 123 ist als eine umlaufende Vertiefung 131 zum Aufnehmen des Kragens 127 ausgebildet.

Das Gehäuse 101, das die elektrische Schaltung 103 umfasst, hat als Gegenstück eine Ausstülpung mit einem Kragen 127. Der Stöpsel 139 wird beim Einsetzen auf den Kragen 127 aufgesetzt. Nach dem Einsetzen der folienartigen Kontaktvorrichtung 105 mit dem Stöpsel 139 ist die Öffnung des Gehäuses 101 abgedichtet. Auf diese Weise kann die Kontaktvorrichtung 105 wasserdicht an der verschlossenen elektrischen Schaltung 103 angesteckt und angebracht werden. Dies vermindert den Aufwand bei der Produktion des oralen Sensorsystems 100 und ermöglicht eine kompakte Bauform und einfache Handhabung.

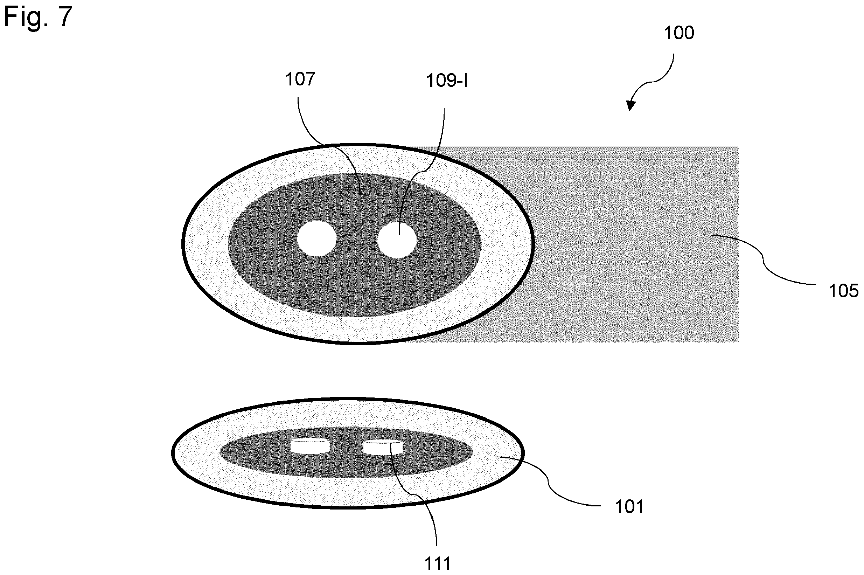

Fig. 7 zeigt eine schematische Ansicht einer weiteren Kontaktvorrichtung 105. Die folienartige Kontaktvorrichtung 105 weist zwei nebeneinander angeordnete innere Kontaktflächen 109-I auf. Die inneren Kontaktflächen 109-I sind von einer Schicht 107 umgeben. Die Schicht 107 besteht aus einem in Verbindung mit Feuchtigkeit quellfähigem und klebendem Material, das Unebenheiten oder eine Beschädigung abdichten kann. Die Schicht 107 läuft um die aufgedruckten Kontaktflächen 109-I der Kontaktvorrichtung 105 herum.

Bei einer Verwendung von Hydrokolloiden in der Schicht 107 quillt die Beschichtung beispielsweise beim Kontakt mit Wasser oder Speichel auf, so dass die dichtende Wirkung erhöht wird. Hydrokolloide gehen in Wasser als Kolloide in Lösung und haben ein hohes Vermögen zur Gelbildung. Die Hydrokolloide sind beispielsweise aus Polysacchariden und Proteinen gebildet. Die klebende und aufquellende Beschichtung damit erzeugt einen Schutz vor eindringender Feuchtigkeit.

Die Schicht 107 wird dadurch wasserdicht mit dem Gehäuse 101 verbunden oder auf das Gehäuse 101 geklebt. Bei jedem Wechsel der Kontaktvorrichtung 105 kann eine unverbrauchte Schicht verwendet werden. Dadurch wird die Dichtigkeit des Überganges zum Gehäuse 101 sichergestellt. Verbrauchte Schichten 107 können bei einer ebenen Anbindungsfläche einfach abgewischt werden. Geringe Rückstände oder Schmutz können mit einer neuen aufquellenden Schicht 107 überbrückt werden. Hierdurch kann ein wasserdichter elektrischer Kontakt zwischen den Kontaktflächen 109-I der Kontaktvorrichtung 105 und den Kontaktflächen 111 der elektrischen Schaltung 103 erreicht werden. Die Klebeverbindung ist so aus einer Beschichtung hergestellt, die kleine Spalte überbrücken kann. Die Kontaktvorrichtung 105 wird durch eine Klebeverbindung auf dem Gehäuse 101 mit der elektrischen Schaltung 103 befestigt.

Die Kontaktvorrichtung 105 ist auf das Gehäuse 101 mit ebener Oberfläche und hervorstehenden Kontakte der elektrischen Schaltung 103 aufgeklebt. Die Kontaktvorrichtung 105 ist aufgrund einer geringen Größe einfach auszuwechseln, ohne dass miniaturisierte mechanische Bauteile benötigt werden. Hierdurch wird eine wasserdichte Verbindung der Kontaktvorrichtung 105 für orale Anwendungen mit der elektrischen Schaltung 103 erreicht. Dies vermindert den Aufwand bei der Produktion des oralen Sensorsystems 100 und ermöglicht eine kompakte Bauform und einfache Handhabung.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Orales Sensorsystem
- 101: Gehäuse
- 103: Schaltung
- 105: Kontaktvorrichtung
- 107: aufquellende Schicht
- 109: Kontaktfläche
- 111: Kontaktfläche
- 113: Dichtungsring
- 115: Aussparung
- 117: Rand
- 119: Sensorbasis
- 121: Dichtmaterial
- 123: Aussparung
- 125: Gehäuseabschnitt
- 127: Kragen
- 129: Schraubgewinde
- 131: Vertiefung
- 133: Klick- oder Schraubmechanismus
- 135: Elastomer-Dichtung
- 137: Lasche
- 139: Stöpsel
- 141: Durchgangskontakt

## Patentansprüche

1. Orales Sensorsystem (100), mit:
- einem Gehäuse (101) mit einer elektrischen Schaltung (103) zum Auswerten von Messsignalen; und
- einer auswechselbaren Kontaktvorrichtung (105) für die elektrische Schaltung (103), die flüssigkeitsdicht mit dem Gehäuse (101) gekoppelt ist.

2. Orales Sensorsystem (100) nach Anspruch 1, wobei die auswechselbare Kontaktvorrichtung (105) eine klebende und/oder aufquellende Schicht (107) zur flüssigkeitsdichten Kopplung mit dem Gehäuse (101) umfasst.

3. Orales Sensorsystem (100) nach Anspruch 2, wobei die klebende und/oder aufquellende Schicht (107) Hydrokolloide umfasst.

4. Orales Sensorsystem (100) nach Anspruch 2 oder 3, wobei die klebende und/oder aufquellende Schicht (107) um die Kontaktflächen (109) herum auf der Kontaktvorrichtung (105) angeordnet ist.

5. Orales Sensorsystem (100) nach einem der vorangehenden Ansprüche, wobei die Kontaktvorrichtung (105) eine Scheibenform, eine Plattenform oder eine Würfelform aufweist.

6. Orales Sensorsystem (100) nach Anspruch 5, wobei die Kontaktvorrichtung (105) auf der einen und/oder anderen Seite Kontaktflächen (111) aufweist.

7. Orales Sensorsystem (100) nach Anspruch 5 oder 6, wobei das Gehäuse (101) eine Aussparung (115) zum Einsetzen der Kontaktvorrichtung (105) aufweist.

8. Orales Sensorsystem (100) nach Anspruch 7, wobei das Gehäuse (101) oder die Kontaktvorrichtung (105) einen Dichtungsring (113) zum Abdichten der Kontaktvorrichtung (105) oder des Gehäuses in der Aussparung (115) umfasst.

9. Orales Sensorsystem (100) nach einem der Ansprüche 5 bis 8, wobei ein Rand (117) der Kontaktvorrichtung (105) aus Kunststoff gebildet ist.

10. Orales Sensorsystem (100) nach einem der Ansprüche 5 bis 9, wobei das Gehäuse (101) und/oder die Kontaktvorrichtung (105) ein Schraubgewinde (129) aufweist.

11. Orales Sensorsystem (100) nach einem der vorangehenden Ansprüche, wobei die Kontaktvorrichtung (105) eine Sensorbasis (119) umfasst, die aus einem Dichtmaterial (121) herausragt.

12. Orales Sensorsystem (100) nach Anspruch 11, wobei das Dichtmaterial (121) eine Aussparung (123) zum Einsetzen eines Gehäuseabschnitts (125) umfasst.

13. Orales Sensorsystem (100) nach Anspruch 12, wobei der Gehäuseabschnitt (125) einen vorstehenden Kragen (127) umfasst.

14. Orales Sensorsystem (100) nach einem der Ansprüche 11 bis 13, wobei die Aussparung (123) eine umlaufende Vertiefung (131) zum Aufnehmen des Kragens (127) umfasst.

15. Orales Sensorsystem (100) nach einem der Ansprüche 11 bis 14, wobei das Dichtmaterial (121) aus Gummi oder Silikon gebildet ist.
